(19) Europäisches Patentamt
European Patent Office
Office européen des brevets

(11) Veröffentlichungsnummer: **0 196 530**
**A2**

(12) # EUROPÄISCHE PATENTANMELDUNG

(21) Anmeldenummer: **86103555.8**

(22) Anmeldetag: **17.03.86**

(51) Int. Cl.⁴: **A61K 31/52**, A61K 31/70, A61K 31/71, A61K 35/72, A23K 1/16, C12P 17/18, C12P 19/28, A61K 31/415, C12P 17/10

(30) Priorität: **26.03.85 CH 1319/85**

(43) Veröffentlichungstag der Anmeldung: **08.10.86 Patentblatt 86/41**

(84) Benannte Vertragsstaaten: **AT BE CH DE FR GB IT LI LU NL SE**

(71) Anmelder: **CTA Finanz AG**
**Guggiweg 8**
**CH-6300 Zug(CH)**

(72) Erfinder: **Moor, Hermann**
**Rheinstrasse 28**
**CH-4302 Augst(CH)**

(74) Vertreter: **Becher, Pauline, Dr. et al**
**A. Braun, Braun, Héritier, Eschmann AG**
**Patentanwälte Holbeinstrasse 36-38**
**CH-4051 Basel(CH)**

(54) **Mittel und Verfahren zur Beschleunigung des Wachstums, zur Optimierung der Fruchtbarkeit und zur Stimulierung des Immunsystems bei Mensch und Tier.**

(57) Das Mittel zur Beschleunigung des Wachstums, zur Optimierung der Fruchtbarkeit und zur Stimulierung des Immunsystems bei Mensch und Tier enthält mindestens einen Wirkstoff, der aus natürlichen Stoffwechselprodukten von Guanin oder Adenin, von Nucleosiden des Guanins oder Adenins, von Nucleotiden des Guanins oder Adenins bzw. von Orotsäure gewählt ist. Als Wirkstoff bevorzugt werden Xanthin, Hypoxanthin, Xanthosin, Inosin, Xanthosinphosphorsäuren, Inosinphosphorsäuren, Orotidinphosphat und Glyoxyldiureid.

Zusätzlich kann das Mittel mindestens einen Wirkstoff enthalten, der aus Oligonucleotiden, deren endständige Basen von Adenin verschieden sind, Nucleinsäurebasen mit Ausnahme von Adenin, Carboxylderivaten von Nucleinsäurebasen mit Ausnahme von Adenin und Aminoderivaten von Nucleinsäurebasen mit Ausnahme von Adenin gewählt ist. Unter Carboxylderivaten bzw. Aminoderivaten werden eine Carboxylgruppe bzw. eine Aminogruppe oder eine zusätzliche Aminogruppe enthaltende Nucleinsäurebasen verstanden.



Rank Xerox

Mittel und Verfahren zur Beschleunigung des Wachstums, zur Optimierung der Fruchtbarkeit und zur Stimulierung des Immunsystems bei Mensch und Tier.

Die bisher in der Vieh-und Geflügelzucht angewendeten wachstumsfördernden Mittel, die sogenannten "Growth Promoter", sind fast ausschliesslich Antibiotika und/oder andere antibakteriell aktive Substanzen, die zum überwiegenden Teil körperfremd sind. Diese Substanzen haben neben der Wachstumsförderung auch die Aufgabe, das in der Massentierhaltung stark erhöhte Risiko von Krankheiten und deren Uebertragung zu begrenzen. Die Nebenwirkungen der genannten Substanzen, das Auftreten von Rückständen im Fleisch und/oder in der Milch und/oder in den Eiern von Nutztieren sowie die Entstehung von Resistenz der Krankheitserreger sprechen aber gegen ihren Einsatz.

Aus der am 2. Mai 1984 unter der Veröffentlichungsnummer 0 107 060 A3 veröffentlichten europäischen Patentanmeldung Nr. 83 11 0325.4 ist bekannt, dass bestimmte Oligonucleotide, Nucleinsäurebasen und Carboxylderivate sowie Aminoderivate von Nucleinsäurebasen dazu verwendet werden können, die Gewebemasse von Organen bei Menschen und Tieren innerhalb ihrer genetischen Schwankungsbreite zu optimieren.

Oligonucleotide und Nucleinsäurebasen sind bekanntlich natürliche Abbauprodukte von Nucleinsäuren. Insbesondere kommen Abbauprodukte von Ribonucleinsäuren, z.B. die Produkte der enzymatischen Spaltung von Hefe-Ribonucleinsäure, in Betracht.

Unter Oligonucleotiden versteht man bekanntlich lineare Sequenzen aus maximal 20 Nucleotiden, in denen die 3'-Position des Zuckers eines Monomers über eine Phosphatgruppe mit der 5'-Position des Zuckers des folgenden Monomers verknüpft ist. Je nach der Kettenlänge unterscheidet man Di-, Tri-, Tetra-, Pentanucleotide usw. Bei den "Monomeren" (Nucleotiden) handelt es sich bekanntlich um Phosphorsäureester von Nucleosiden. Nucleoside sind bekanntlich N-Glykoside von Nucleinsäurebasen. Als Zuckerkomponente der Glykoside spielen besonders D-Ribose und D-2-Desoxyribose in der Furanosekonfiguration eine Rolle.

Zu den Nucleinsäurebasen zählen Purin-und Pyrimidinbasen sowie seltene Nucleinsäurebausteine; die seltenen Nucleinsäurebausteine entstehen durch enzymatisch katalysierte Modifizierungen der üblichen Nucleinsäurebasen Adenin, Guanin, Cytosin, Uracil und Thymin. Diese üblichen Nucleinsäurebasen sind Purinderivate, nämlich Adenin (6-Aminopurin) der Formel:

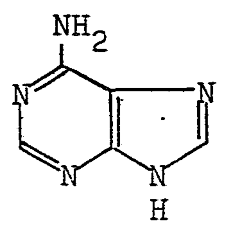

und Guanin (2-Amino-6-hydroxypurin) der Formel:

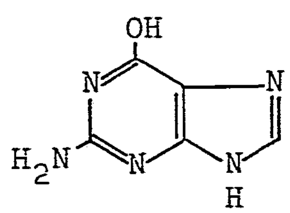

sowie Pyrimidinderivate, nämlich Cytosin (2-Hydroxy-6-aminopyrimidin) der Formel:

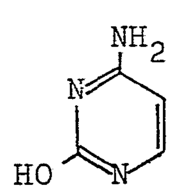

Uracil (2,4-Dihydroxypyrimidin) der Formel:

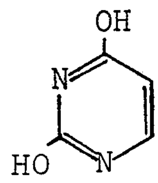

und Thymin (2,6-Dihydroxy-5-methylpyrimidin) der Formel:

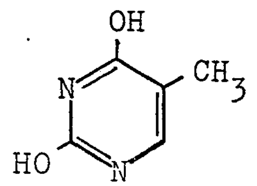

Mit Ausnahme der Formel des Adenins enthalten alle obigen Formeln mindestens eine einer Doppelbindung benachbarte Hydroxylgruppe, wobei diese Hydroxylgruppen im Gleichgewicht mit Oxogruppen (Keto-Enol-Gleichgewicht) stehen. Dies sei am Beispiel des Guanins bzw. Cytosins erläutert:

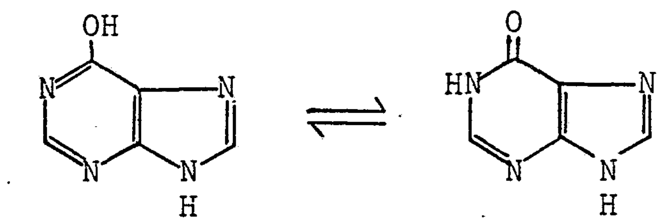

Guanin

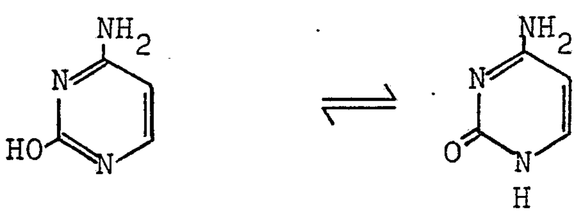

Cytosin

Es hatte sich nun gezeigt, dass nur Nucleinsäurebasen, die im Ringsystem mindestens eine einer Doppelbindung benachbarte Hydroxylgruppe enthalten, deren Carboxyl-und Aminoderivate sowie solche Nucleinsäurebasen als endständige Basen enthaltende Oligonucleotide für die Zwecke der oben erwähnten europäischen Patentanmeldung geeignet sind. Deshalb wurde die Verwendung von Adenin, von seinen Carboxyl-und Aminoderivaten sowie von Adenin als endständige Basen enthaltenden Oligonucleotiden als Wirkstoffe ausgeschlossen.

Es war nun überraschend, dass diese nicht aktiven Nucleinsäurebausteine zum Beispiel beim Nachfermentieren mit Hefen bzw. beim Autolysieren von Hefen, denen sie als Stickstoffquellen gedient hatten, in aktive Verbindungen übergingen. In die Abbauprodukte der inaktiven Adeninkörper war dabei durch oxydative Desaminierung eine einer Doppelbindung benachbarte Hydroxylgruppe eingeführt worden. Dies sei am Beispiel des Adenins erläutert:

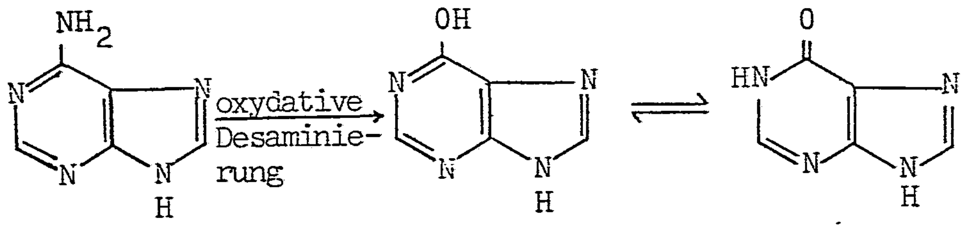


Adenin

Im Stoffwechsel, beispielsweise im Hefestoffwechsel, werden auf diese Weise z.B. Guanin in Xanthin (2,6-Dihydroxypurin), Adenin in Hypoxanthin (6-Hydroxypurin). Guanosin (ein β-glykosidisches Nucleosid aus D-Ribose und Guanin) in Xanthosin (ein β-glykosidisches Nucleosid aus D-Ribose und Xanthin), Adenosin (ein β-glykosidisches Nucleosid aus D-Ribose und Adenin) in Inosin (ein β-glykosidisches Nucleosid aus D-Ribose und Hypoxanthin), Guanylsäure (Guanosin-5'-monophosphat) in Xanthylsäure (Xanthosin-5'-monophosphat) und Adenylsäure (Adenosin-5'-monophosphat) in Inosinsäure (Inosin-5'-monophosphat) übergeführt. Allen diesen durch oxydative Desaminierung entstandenen Stoffwechselprodukten ist gemeinsam, dass sie eine einer Doppelbindung benachbarte Hydroxylgruppe mehr haben als das Ausgangsmaterial und deshalb für den Erfindungszweck wirksam bzw. wirksamer sind. Neben diesen Desaminierungsreaktionen werden auch Purinkörper über Harnsäure in Glyoxyldiureid übergeführt und wird Orotsäure (Uracil-4-carbonsäure) in Orotidin-5'-monophosphat übergeführt (Orotidin ist ein β-glykosidisches Nucleosid aus D-Ribose und Orotsäure). Alle diese Verbindungen sind überraschenderweise einzeln und im Gemisch miteinander brauchbar zur Beschleunigung des Wachstums, zur Optimierung der Fruchtbarkeit und zur Stimulierung des Immunsystems bei Mensch und Tier.

Durch die Nachfermentation und/oder oxydative Desaminierung von Produkten der enzymatischen Spaltung von Ribonucleinsäuren, z.B. Hefe-Ribonucleinsäure, die z.B. mit Hefe erfolgen kann, können daher die Wirkstoffausbeuten wesentlich (z.B. um etwa ein Drittel) erhöht werden, was natürlich die Wirtschaftlichkeit des Verfahrens signifikant verbessert.

Ferner wurde durch Tierversuche festgestellt, dass Gemische von Orotsäure oder Orotidinphosphat mit Glyoxyldiureid einen QAB-Effekt (QAB = Quick Antibody Builder) haben, das heisst, sie stimulieren die Bildung von Antikörper erzeugenden Zellen, Orotsäure oder Orotidinphosphat einerseits und Glyoxyldiureid andererseits können in Gewichtsverhältnissen von 1:2 bis 1:20 verwendet werden. Grössere Mengen Glyoxyldiureid sind denkbar, bringen aber keine wesentlichen Verbesserungen hervor.

Ein Tierversuch wurde folgendermassen durchgeführt:

Eine Mischung aus 1 g Orotidinphosphat und 2 g Glyoxyldiureid wurde einer Untersuchung über den Einfluss auf eine T-zellabhängige B-Zellreaktion (Bildung von Plasmazellen) nach Gabe von Schaferythrozyten (SRBC) an Mäusen unterworfen.

a) BALB/c-Mäuse

Dosierung: 10 mg/kg Körpergewicht p.o.

Bei einer Dosierung von 10 mg/kg Körpergewicht p.o. kommt es zu einem statistisch signifikanten ($p<0,001$) Anstieg der Antikörper erzeugenden Milzzellen.

b)Swiss-Mäuse

Dosierung: 10 mg/kg Körpergewicht p.o.

Es findet eine statistisch signifikante ($p<0,01$) Zunahme der Antikörper erzeugenden Milzzellen statt.

Diese Befunde zeigen eine immunstimulierende Wirkung der Prüfsubstanz.

Die Erfindung bezieht sich daher auf ein Mittel zur Beschleunigung des Wachstums, zur Optimierung der Fruchtbarkeit und zur Stimulierung des Immunsystems bei Mensch und Tier, das mindestens einen Wirkstoff enthält, der aus natürlichen Stoffwechselprodukten von Guanin oder Adenin, von Nucleosiden des Guanins oder Adenins, von Nucleotiden des Guanins oder Adenins bzw. von Orotsäure gewählt ist. Vorzugsweise sind die Wirkstoffe aus Xanthin, Hypoxanthin, Xanthosin, Inosin, Xanthosinphosphorsäuren, Inosinphosphorsäuren, Orotidinphosphat und Glyoxydiureid gewählt.

Zusätzlich kann das Mittel mindestens einen weiteren Wirkstoff enthalten, der aus Oligonucleotiden, deren endständige Basen von Adenin verschieden sind, Nucleinsäurebasen mit Ausnahme von Adenin, Carboxylderivaten von Nucleinsäurebasen mit Ausnahme von Adenin und Aminoderivaten von Nucleinsäurebasen mit Ausnahme von Adenin gewählt ist.

Besonders bevorzugt wird ein solches Mittel, das Orotidinphosphat oder Orotsäure einerseits und Glyoxyldiureid andererseits in einem Gewichtsverhältnis von 1:2 bis 1:20 enthält, für die Immunstimulierung.

Die erfindungsgemässen Mittel können Konzentrate oder Dosier-Einheiten sein, die 1 bis 200 mg, vorzugsweise 30 bis 150 mg, insbesondere ca. 80 mg Wirkstoff(e) pro g enthalten. Es kann sich auch um ein gebrauchsfertiges Futtermittel oder Trinkwasser handeln, das 1 bis 200 g, vorzugsweise 30 bis 150 g, insbesondere ca. 8o g Wirkstoff(e) pro 1000 kg enthält.

Ferner bezieht sich die Erfindung auf ein Verfahren zur Beschleunigung des Wachstums, zur Optimie rung der Fruchtbarkeit und zur Stimulierung des Immunsystems bei Mensch und Tier, das dadurch gekennzeichnet ist, dass man mindestens einen Wirkstoff, der aus natürlichen Stoffwechselprodukten von Guanin oder Adenin, von Nucleosiden des Guanins oder Adenins, von Nucleotiden des Guanins oder Adenins bzw. von Orotsäure gewählt ist, in den menschlichen oder tierischen Körper einbringt. Vorzugsweise sind die Wirkstoffe aus Xanthin, Hypoxanthin, Xanthosin, Inosin, Xanthosinphosphorsäuren, Inosinphosphorsäuren, Orotidinphosphat und Glyoxyldiureid gewählt.

Gemäss einer weiteren Ausführungsform der Erfindung kann man mindestens einen der oben genannten Wirkstoffe im Gemisch mit mindestens einem weiteren Wirkstoff, der aus Oligonucleotiden, deren endständige Basen von Adenin verschieden sind, Nucleinsäurebasen mit Ausnahme von Adenin, Carboxylderivaten von Nucleinsäurebasen mit Ausnahme von Adenin und Aminoderivaten von Nucleinsäurebasen mit Ausnahme von Adenin gewählt ist, in den menschlichen oder tierischen Körper einbringen.

Zur Immunstimulierung verwendet man vorzugsweise ein Gemisch, das Orotidinphosphat oder Orotsäure einerseits und Glyoxyldiureid andererseits in einem Gewichtsverhältnis von 1:2 bis 1:20 enthält.

Man kann den Wirkstoff bzw. die Wirkstoffe durch Infusion oder Injektion oder per os, z.B. bei Tieren mit dem Futter oder Trinkwasser, verabreichen. Man kann z.B. 0,02 bis 10 mg, vorzugsweise 2 bis 6 mg, insbesondere ca. 3 mg Wirkstoff(e) pro kg Körpergewicht und Tag verabreichen.

Herstellungsbeispiel

Stufe 1:

Im Handel erhältliche Ribonucleinsäure wird in Wasser suspendiert, so dass eine Suspension mit ca. 10 % Trockensubstanz entsteht. Es wird so viel Salzsäure zugesetzt, dass der flüssige Anteil der Suspension 1 Mol HC1 pro Liter enthält. Die angesäuerte Suspension wird eine Stunde lang am Rückfluss gekocht und abgekühlt. Der gebildete Niederschlag, der aus den Hydrochloriden des Adenins und des Guanins besteht, wird abgetrennt.

Stufe 2:

Ein virulenter Stamm von Torula-Hefe wird in üblicher Weise kultiviert mit der Ausnahme, dass die in Stufe 1 hergestellten Purinbasen als einzige Stickstoffquellen zugesetzt werden. Schon vom zweiten Generationszyklus an - (nach 4 bis 6 Stunden) kann eine für die vorliegenden Zwecke geeignete Hefe geerntet werden. Die Hefekultur wird zentrifugiert, bis ein zur Weiterverarbeitung geeignetes Zentrifugat mit ca. 15 % Trockensubstanz erhalten wird. Diese Hefe erzeugt Enzyme, die Adenin und Guanin spezifisch oxydativ desaminieren, und kann in der Stufe 3 verwendet werden. Gewünschtenfalls kann die reine Torula-Hefe durch Backhefe oder Bierhefe usw. ersetzt werden.

Stufe 3:

Eine Suspension von industriell erzeugter Hefe (Torula-Hefe, Backhefe, Bierhefe usw.), die ca. 15 % Trockensubstanz enthält, wird in einen wärmeisolierten, heizbaren, mit einem Rührwerk versehenen Tank eingefüllt und auf Gärtemperatur gebracht. Dann wird frische Nährlösung, in der 1 % Orotsäure und 1 % Nikotinsäure, bezogen auf die Hefe-Trockensubstanz, suspendiert worden sind, in einer Menge, die für ungefähr einen Generationszyklus ausreicht, in den Tank gegeben. Das Hefe-Zentrifugat, das in Stufe 2 erhalten wurde, wird in einem Gewichtsverhältnis von 1 : 7, bezogen auf die Trockensubstanz, zu der Hefesuspension in dem Tank zugesetzt. Unter Rühren wird 2,5 Stunden lang fermentiert. Danach wird eine Suspension von im Handel erhältlicher Ribonucleinsäure, die 20 % Trockensubstanz enthält, in einer Menge zugesetzt, die 2 Gewichtsteilen Ribonucleinsäure-Trockensubstanz pro Gewichtsteil Hefe-Trockensubstanz entspricht.

Stufe 4:

In dieser Stufe wird ein erfindungsgemässes Mittel, das monomere oder kurzkettige Nucleinsäurekomponenten enthält, durch Autolyse von Hefe und Hydrolyse von Ribonucleinsäure in Gegenwart der aus den Hefezellen freigesetzten Enzyme hergestellt. Dieses Mittel eignet sich besonders für die Optimierung von Drüsen-und Embryonalgewebe, speziell bei Zuchttieren.

Die in Stufe 3 hergestellte, zugesetzte Ribonucleinsäure enthaltende Hefesuspension wird unter Rühren langsam erwärmt, so dass die Temperatur innerhalb von 2 Stunden 55°C erreicht. Dann wird der pH-Wert mit Natriumhydroxyd auf 6 gebracht. Nun setzt man 0,1 % Papain und 0,2 % Bakterienprotease, bezogen auf die Hefe-Trockensubstanz, zu und lässt 12 Stunden lang bei 55°C autolysieren. Dann wird das Gemisch aufgekocht und getrocknet. Der Nucleinsäurebasengehalt und der Hemmtiter gegen L-Aminosäureoxydase dieses Produkts werden bestimmt.

**Ansprüche**

1) Mittel zur Beschleunigung des Wachstums, zur Optimierung der Fruchtbarkeit und zur Stimulierung des Immunsystems bei Mensch und Tier, dadurch gekennzeichnet, dass es mindestens einen Wirkstoff enthält, der aus natürlichen Stoffwechselprodukten von Guanin oder Adenin, von Nucleosiden des Guanins oder Adenins, von Nucleotiden des Guanins oder Adenins bzw. von Orotsäure gewählt ist.

2) Mittel nach Anspruch 1, dadurch gekennzeichnet, dass es mindestens einen Wirkstoff enthält, der aus Xanthin, Hypoxanthin, Xanthosin, Inosin, Xanthosinphosphorsäuren, Inosinphosphorsäuren, Orotidinphosphat und Glyoxyldiureid gewählt ist.

3) Mittel nach Anspruch 1 oder 2, dadurch gekennzeichnet, dass es zusätzlich mindestens einen weiteren Wirkstoff enthält, der aus Oligonucleotiden, deren endständige Basen von Adenin verschieden sind, Nucleinsäurebasen mit Ausnahme von Adenin, Carboxylderivaten von Nucleinsäurebasen mit Ausnahme von Adenin und Aminoderivaten von Nucleinsäurebasen mit Ausnahme von Adenin gewählt ist.

4) Mittel zur Immunstimulierung nach einem der Ansprüche 1 bis 3, dadurch gekennzeichnet, dass es Orotidinphosphat oder Orotsäure einerseits und Glyoxyldiureid andererseits in einem Gewichtsverhältnis von 1:2 bis 1:20 enthält

5) Mittel nach einem der Ansprüche 1 bis 4, dadurch gekennzeichnet, dass es ein Konzentrat oder eine Dosier-Einheit ist, das bzw. die 1 bis 200 mg, vorzugsweise 30 bis 150 mg, insbesondere ca. 80 mg Wirkstoff(e) pro g enthält.

6) Mittel nach einem der Ansprüche 1 bis 4, dadurch gekennzeichnet, dass es ein gebrauchsfertiges Futtermittel oder Trinkwasser ist, das 1 bis 200 g, vorzugsweise 30 bis 150 g, insbesondere ca. 80 g Wirkstoff(e) pro 1000 kg enthält.

7) Verfahren zur Herstellung eines Mittels nach Anspruch 1, dadurch gekennzeichnet, dass man

a) einen virulenten Hefestamm in üblicher Weise kultiviert,

aber als einzige Stickstoffquelle Adenin und/oder Guanin verwendet,

b) die Hefekultur aus Stufe a) im zweiten Generationszyklus zentrifugiert, bis das Zentrifugat ca. 15% Trokkensubstanz enthält,

c) eine Suspension von frischer Hefe, die ca. 15 % Trokkensubstanz enthält, auf Gärtemperatur erwärmt,

d) zu der Suspension aus Stufe c) frische Nährlösung, die 1 % Orotsäure und 1 % Nicotinsäure, bezogen auf die Hefe-Trockensubstanz, enthält, in einer Menge zusetzt, die für ungeführ einen Generationszyklus ausreicht,

e) zu der Suspension aus Stufe d) das Hefezentrifugat aus Stufe b) in einem Gewichtsverhältnis von 1:7, bezogen auf die Trockensubstanz, zusetzt,

f) das Gemisch aus Stufe d) unter Rühren 2,5 Stunden lang fermentiert,

g) zu der fermentierten Suspension aus Stufe f) eine Suspension von Ribonucleinsäure, die 20 % Trockensubstanz enthält, in einer Menge zusetzt, die 2 Gewichtsteilen Ribonucleinsäure-Trockensubstanz pro Gewichtsteil Hefe-Trockensubstanz entspricht,

h) die zugesetzte Ribonucleinsäure enthaltende Hefesuspension aus Stufe g) unter Rühren langsam auf 55 °C erwärmt,

i) den pH-Wert der Hefesuspension aus Stufe h) mit Natriumhydroxyd auf 6 bringt,

j) zu der Hefesuspension aus Stufe i) 0,1 % Papain und 0,2 % Bakterienprotease, bezogen auf die Hefe-Trockensubstanz, zusetzt,

k) die Hefesuspension aus Stufe j) 12 Stunden lang bei 55 °C autolysieren lässt und

l) das Gemisch aus Stufe k) aufkocht und trocknet.

8) Verfahren zur Beschleunigung des Wachstums, zur Optimierung der Fruchtbarkeit und zur Stimulierung des Immunsystems bei Mensch und Tier, dadurch gekennzeichnet, dass man mindestens einen Wirkstoff, der aus natürlichen Stoffwechselprodukten von Guanin oder Adenin, von Nucleosiden des Guanins oder Adenins, von Nucleotiden des Guanins oder Adenins bzw. von Orotsäure gewählt ist, in den menschlichen oder tierischen Körper einbringt.

9) Verfahren nach Anspruch 8, dadurch gekennzeichnet, dass man mindestens einen Wirkstoff verwendet, der aus Xanthin, Hypoxanthin, Xanthosin, Inosin, Xanthosinphosphorsäuren, Inosinphosphorsäuren, Orotidinphosphat und Glyoxyldiureid gewählt ist.

10) Verfahren nach Anspruch 8 oder 9, dadurch gekennzeichnet, dass man mindestens einen der im Anspruch 7 bzw. 8 genannten Wirkstoffe im Gemisch mit mindestens einem weiteren Wirkstoff verwendet, der aus Oligonucleotiden, deren endständige Basen von Adenin verschieden sind, Nucleinsäurebasen mit Ausnahme von Adenin, Carboxylderivaten von Nucleinsäurebasen mit Ausnahme von Adenin und Aminoderivaten von Nucleinsäurebasen mit Ausnahme von Adenin gewählt ist.

11) Verfahren nach einem der Ansprüche 8 bis 10 zur Immunstimulierung, dadurch gekennzeichnet, dass man ein Gemisch verwendet, das Orotidinphosphat oder Orotsäure einerseits und Glyoxyldiureid andererseits in einem Gewichtsverhältnis von 1:2 bis 1:20 enthält.

12) Verfahren nach einem der Ansprüche 8 bis 11, dadurch gekennzeichnet, dass man den bzw. die Wirkstoff(e) durch Infusion oder Injektion oder per os, z.B. bei Tieren mit dem Futter oder Trinkwasser, verabreicht.

13) Verfahren nach einem der Ansprüche 8 bis 12, dadurch gekennzeichnet, dass man 0,02 bis 10 mg, vorzugsweise 2 bis 6 mg, insbesondere ca. 3 mg Wirkstoff(e) pro kg Körpergewicht und Tag verabreicht.